# EUROPÄISCHE PATENTSCHRIFT

(11) **EP 3 621 562 B1**
(45) Veröffentlichungstag und Bekanntmachung des Hinweises auf die Patenterteilung: **20.04.2022**
(21) Anmeldenummer: 18723816.7
(22) Anmeldetag: 08.05.2018
(51) Int. Cl.: A61F 5/01

(54) **FUSSBEWEGUNGSDÄMPFUNGSVORRICHTUNG UND SCHUH ZUM DÄMPFEN EINER FUSSBEWEGUNG ÜBER DAS SPRUNGGELENK**
FOOT MOVEMENT DAMPER AND SHOE FOR DAMPING A FOOT MOVEMENT VIA THE ANKLE JOINT
DISPOSITIF D'AMORTISSEMENT DU MOUVEMENT DU PIED ET CHAUSSURE DESTINÉE À AMORTIR UN MOUVEMENT DU PIED PAR LE BIAIS DE LA CHEVILLE

(30) Priorität: 08.05.2017 DE 102017109877
(43) Veröffentlichungstag der Anmeldung: 18.03.2020
(73) Patentinhaber: Betterguards Technology GmbH, 10625 Berlin (DE)
(72) Erfinder: BICHLER, Vinzenz, 10777 Berlin (DE); STUMPER, Timo, 10963 Berlin (DE); BUSCHINGER, Oscar, 10707 Berlin (DE)
(74) Vertreter: Okoampah, Rene
(86) Internationale Anmeldenummer: PCT/EP2018/061933
(87) Internationale Veröffentlichungsnummer: WO 2018/206611

(56) Entgegenhaltungen:
- EP-A1- 0 824 014
- DE-A1-102012 011 433
- DE-A1-102015 107 405

## Beschreibung

### Technisches Gebiet

Die vorliegende Erfindung betrifft eine Fußbewegungsdämpfungsvorrichtung zum Dämpfen einer Fußbewegung über das Sprunggelenk und einen entsprechend ausgebildeten Schuh zum Dämpfen einer Fußbewegung über das , Sprunggelenk, wie in den Ansprüchen definiert.

### Stand der Technik

Es ist bekannt, die Bewegung des Sprunggelenks mittels Vorrichtungen zum Begrenzen von Fußbewegungen zu stabilisieren, um Traumata infolge eines Umknickens, das heißt einer Bewegung des Sprunggelenks über zumindest eines seiner Sprunggelenksachsen in einem unphysiologischen Bereich entgegenzuwirken. Die häufigste Form des Umknicktraumas ist eine Sprunggelenksdistorsion als Dehnung oder Ruptur infolge einer Inversion. Inversionsbewegungen über eine Sprunggelenksachse resultieren aufgrund des ansteigenden Inversionswinkels in einer Änderung des Abstands zwischen Fuß und Unterschenkel. Bei Überschreiten eines bestimmten Inversionswinkels oder einer bestimmten Inversionsgeschwindigkeit beziehungsweise Inversionsbeschleunigung kann es zu Verletzungen des Bandapparats des Sprunggelenks oder zu Rupturen kommen.

Um dies zu verhindern, sind Vorrichtungen bekannt, die in einem bestimmten Umfang Bewegungen ermöglichen und ab einem bestimmten Grenzwinkel der Bewegung um die Sprunggelenksachse eine Bewegung gänzlich verhindern. Hierzu sind einerseits relativ starren Orthesen bekannt, bei denen das Hemmen von Bewegungen unter Verwendung von Schienen oder Schienenplatten im Vordergrund steht.

Ferner sind Vorrichtungen bekannt, bei welchen eine Bewegung des Sprunggelenks bis zu einem bestimmten Grenzwinkel der Bewegung zugelassen wird, und aufgrund des Aufbaus ab diesem Grenzwinkel eine Bewegung gänzlich blockiert wird. Solch eine Vorrichtung ist beispielsweise aus der EP 2717809 B1 bekannt. Solche Vorrichtungen bieten vor Erreichen des Grenzwinkels keinerlei Schutzwirkung. Es ist jedoch bekannt, dass das Verletzungsrisiko beim Umknicken, insbesondere bei bereits geschwächten Bändern oder nach einer Verletzung, jedoch auch signifikant von der beim Umknicken auftretenden Inversionsgeschwindigkeit und Inversionsbeschleunigung und nicht allein vom Inversionswinkel abhängt. Im Bereich unterhalb des Grenz(inversions)winkels bieten solche Vorrichtungen keinerlei Schutz. Zudem ist nach Erreichen des Grenzwinkels die Bewegung komplett blockiert. Das abrupte Abstoppen der Umknickbewegung erzeugt eine große Belastung auf die Struktur des Sprunggelenks, wodurch die Gefahr einer Verletzung, beispielsweise eine Ruptur am Knochen oder eine Knorpelverletzung erhöht wird. Durch dieses Blockieren des Sprunggelenks wird zudem die Umknickbewegung auf das nächste Gelenk übertragen. Bei einem Umknicken über das Sprunggelenk ist dies das Kniegelenk. Aufgrund des vorliegenden großen Hebelarms, der ungünstigen Krafteinleitung und der Komplexität des Kniegelenks kann es dann zu schwerwiegenden Verletzungen, wie Kreuzbandrissen oder Meniskusschäden führen, welche sich aufgrund ihrer Einschränkung der Person, der Komplexität und der weniger guten Heilungsmöglichkeiten vielfach negativer Auswirken, als eine Sprunggelenksbänderverletzung. Ferner sind Vorrichtungen bekannt, welche im angezogenen Zustand stets ein Mindestmaß an Bewegung zulassen, bei gefährlichen Bewegungen jedoch blockieren. Aus der DE 10 2014 107 335 A1 ist eine Vorrichtung zum adaptiven Begrenzen einer Inversionsbewegung über das Sprunggelenk gezeigt. Die darin gezeigte Sprunggelenksorthese benötigt, um zu verhindern, dass sich der obere Teil der Orthese bei einer Krafteinwirkung vom Fuß kommend in Richtung Fuß verschiebt, zur Abstützung der Aufnahme des oberen Bereichs einen auf der medialen Seite des Sprunggelenks gelegenen druckstabilen Balken, welcher sich zwischen dem oberen Bereich der Orthese und dem unteren Bereich der Orthese erstreckt und fest mit beiden Bereichen verbunden sein muss. Der Balken muss dabei derart ausgebildet sein, dass er die über die Auszugsvorrichtung auf der lateralen Seite in den oberen Bereich eingeleiteten Zugkräfte als Druckkräfte auf der medialen Seite wieder in den unteren Bereich zurückleitet, ohne sich zu Verformen und zu knicken. Dadurch kann zwar eine Positionssicherung in distaler Richtung erzielt werden. Jedoch beschränkt der Balken auf der medialen Seite des Fußes weiterhin die Bewegungsfreiheit des Sprunggelenks. DE102012011433A1 zeigt eine Vorrichtung mit dilatantem Material zur adaptiven Bewegungsbegrenzung. EP0824014A1 zeigt eine Bandage zur Fixierung des Sprunggelenks. DE102015107405A1 zeigt eine Vorrichtung zum Begrenzen einer Bewegung eines zwischen einem ersten Körperteil und einem zweiten Körperteil angeordneten Gelenks.

### Darstellung der Erfindung

Ausgehend von dem bekannten Stand der Technik ist es eine Aufgabe der vorliegenden Erfindung, eine verbesserte Fußbewegungsdämpfungsvorrichtung zum Dämpfen einer Fußbewegung über das Sprunggelenk bereitzustellen.

Die Aufgabe wird durch eine Fußbewegungsdämpfungsvorrichtung zum Dämpfen einer Fußbewegung über das Sprunggelenk mit den Merkmalen des Anspruchs 1 gelöst. Vorteilhafte Weiterbildungen ergeben sich aus den Unteransprüchen, der Beschreibung und den Figuren.

Entsprechend wird eine Fußbewegungsdämpfungsvorrichtung zum Dämpfen einer Fußbewegung über das Sprunggelenk vorgeschlagen, umfassend eine Stützanordnung zum Abstützen an einem Unterschenkel und eine Halteanordnung zum Halten an einem Fuß. Erfindungsgemäß ist eine Dämpfungsanordung mit mindestens einem Dämpfungselement zum Dämpfen einer Relativbewegung zwischen der Stützanordnung und der Halteanordnung angeordnet, wobei die Dämpfungsanordnung eine Stützanbindung zum Anbinden an die Stützanordnung und eine Halteanbindung zum Anbinden an die Halteanordnung aufweist. Die Stützanordnung umfasst ferner zumindest einen Abstützbereich und mindestens eine Abstützfixierung, wobei sich die Stützanordnung mit dem zumindest einen Abstützbereich oberhalb des Knöchels des Sprunggelenks an dem Knöchel abstützen kann, und wobei die mindestens eine Abstützfixierung zum Fixieren der Position des zumindest einen Abstützbereichs in einem an den Unterschenkel angebrachten Zustand ausgebildet ist.

Der Begriff "Sprunggelenk" umfasst vorliegend das obere und das untere Sprunggelenk und entsprechend die Bewegungsachse des oberen Sprunggelenks, welche im Wesentlichen die Plantarflexion und Dorsalextension des Fußes ermöglicht, und die Bewegungsachse des unteren Sprunggelenks, welche im Wesentlichen die Inversion und Eversion, umfassend Supination, Adduktion und Plantarflexion sowie Abduktion und Dorsalextension, ermöglicht.

Unter dem Begriff "Knöchel" wird hier die Ausprägung der Gelenkpfanne, der Malleolengabel des oberen Sprunggelenks verstanden. Folglich umfasst der Begriff "Knöchel" vorliegend den Außenknöchel, welcher durch die Ausprägung des Malleolus lateralis ausgebildet ist, und den Innenknöchel, welcher durch die Ausprägung des Malleolus medialis ausgebildet ist. Aufgrund der Ausbildung der Malleolengabel als Gelenkpfanne weist der Knöchel im Vergleich zum sich oberhalb, folglich proximal anschließenden Abschnitt des Unterschenkel in Bezug auf die proximaldistale Richtung eine größere Querschnittsfläche auf. Mithin ist der Umfang des Knöchels im Vergleich zum sich proximal anschließenden Abschnitt größer.

Dadurch, dass eine Dämpfungsanordung mit mindestens einem Dämpfungselement zum Dämpfen einer Relativbewegung zwischen der Stützanordnung und der Halteanordnung vorgesehen ist, wobei die Dämpfungsanordnung eine Stützanbindung zum Anbinden an die Stützanordnung und eine Halteanbindung zum Anbinden an die Halteanordnung aufweist, kann eine Bewegung auch über einen Grenzwinkel der Bewegung des Sprunggelenks bereitgestellt werden. Zudem kann die Bewegung bei jeder Stellung des Fußes gedämpft werden.

Dadurch, dass die Stützanordnung zumindest einen Abstützbereich und mindestens eine Abstützfixierung umfasst, wobei sich die Stützanordnung mit dem zumindest einen Abstützbereich oberhalb des Knöchels des Sprunggelenks an dem Knöchel abstützen kann, und wobei die mindestens eine Abstützfixierung zum Fixieren der Position des zumindest einen Abstützbereichs in einem an den Unterschenkel angebrachten Zustand ausgebildet ist, ist sichergestellt, dass bei einer Zugkrafteinwirkung auf die Stützanordnung über die Dämpfungsanordnung in Richtung der Halteanordnung die Kraft von der Stützanordnung über deren mindestens einen Abstützbereich auf den Knöchel übertragen werden kann. Mit anderen Worten stützt sich die Stützanordnung im angelegten beziehungsweise angezogenen Zustand der Fußbewegungsdämpfungsvorrichtung am Benutzer von oben, das heißt proximal, in Richtung des Fußes, das heißt distal, auf den Knöchel im Wesentlichen formschlüssig ab. Der Formschluss wird dabei durch das Umschließen des verjüngten Bereiches des Unterschenkels proximal des Knöchels ermöglicht. Ferner ist es somit möglich, die Stützanordnung flexibel zu gestalten. Eine starre Anordnung mit einer harten Schale, welche den Knöchel beziehungsweise das Sprunggelenk flächig bedeckt, ist dadurch nicht notwendig.

Die Abstützfixierung ist dabei so ausgebildet, dass sie den mindesten einen Abstützbereich im angelegten beziehungsweise angezogenen Zustand von einer Lageänderung bezüglich des Unterschenkels hindert. Bevorzugt ist die Abstützfixierung dabei als zugsteifes Band, bevorzugt als Klettband, mit einer Umlenköse und einem Klettverschluss ausgebildet, so dass sie über die Umlenköse am Unterschenkel straff gezogen und mittels des Klettverschlusses fixiert werden kann. Insbesondere wird dadurch ein radiales Aufweiten der Stützanordnung verhindert, so dass der mindestens eine Abstützbereich nicht über den Knöchel nach unten, das heißt distal, rutschen kann. Alternativ kann die Abstützfixierung auch als Schnürung an der Stützanordnung bereitgestellt sein.

Insbesondere durch die Kombination aus dem vorgenannten Vorsehen der Dämpfungsanordnung zwischen Stützanordnung und Halteanordnung und der vorgenannten Ausbildung der Stützanordnung kann so eine hohe Beweglichkeit des Sprunggelenks bei physiologischen Bewegungen, welche keine Verletzungen hervorrufen, und gleichzeitig eine gute Schutzwirkung bei unphysiologischen Bewegungen bei jeder Stellung des Fußes erzielt werden, ohne das Kniegelenk beim Dämpfen der Umknickenbewegung durch die Fußbewegungsdämpfungsvorrichtung über Gebühr und zu beanspruchen. Durch das mindestens eine Dämpfungselement kann ein Teil der beim Umknicken entstehenden Energie absorbiert werden, welche ansonsten bei vergrößertem Hebelarm durch das Kniegelenk abgefangen werden müsste. Das mindestens eine Dämpfungselement ist dabei bevorzugt so eingestellt, dass eine hinreichend große Dämpfungswirkung zum Vermeiden von Verletzungen am Sprunggelenk bereitgestellt ist, und zudem genügend Energie absorbiert werden kann, um Verletzungen auch am Kniegelenk zu vermeiden. Die beim Umknicken entstehende Energie wird so teilweise durch das mindestens eine Dämpfungselement absorbiert und über die Stützanordnung von oberhalb des Knöchels über den oberen Bereich der äußeren Ausprägung der Malleolengabel in den Unterschenkel eingeleitet, sowie teilweise durch das Kniegelenk ausgeglichen.

In einer bevorzugten Weiterführung weist die Stützanordnung flexible, biegsame Materialien auf. Bevorzugt ist der mindestens eine Abstützbereich durch im angezogenen Zustand am Unterschenkel anliegenden Schaumstoff in Kombination mit einem radial außen an dem Schaumstoff angeordneten, in Umfangsrichtung des Unterschenkels verlaufenden zugsteifen Band ausgebildet. Bevorzugt bildet letzteres gleichzeitig die Abstützfixierung. Dadurch ist es möglich, die Stützanordnung als flexible Fußfessel auszugestalten. Aufgrund der Flexibilität der Stützanordnung kann vermieden werden, dass es am Knöchel zu lokalen Druckstellen oder Scheuerstellen kommt. Der mindestens eine Abstützbereich schmiegt sich aufgrund der flexiblen Struktur an die Oberfläche der Oberseite des Knöchels an und erzeugt so eine gleichmäßige Druckverteilung.

In einer Weiterführung weist die Stützanordnung bevorzugt lokal angeordnete, formstabile beziehungsweise druckstabile Elemente auf, über welche die in die Stützanordnung eingeleitete Kraft gleichmäßig auf den Knöchel übertragen werden können.

In einer weiteren bevorzugten Ausführungsform weist die Stützanordnung zumindest einen ersten Abstützbereich zum Abstützen an einer ersten Seite, bevorzugt einer lateralen Seite des Sprunggelenks, und zumindest einen zweiten Abstützbereich zum Abstützen an einer an einer zweiten Seite, bevorzugt einer medialen Seite des Sprunggelenks auf. Dadurch kann ein gleichmäßiges Abstützen der Stützanordnung auf dem Knöchel erzielt werden. Da hierdurch die von der Halteanordnung über die Dämpfungsanordnung kommenden Kräfte über die Mehrzahl an Abstützbereichen verteilt in den Knöchel übertragen werden und so eine große, einseitig beziehungsweise lokal eingeleitete Kraft vermieden wird, kann die Stützanordnung besonders sicher in ihrer Position gehalten werden. Wenn der erste Abstützbereich derart ausgebildet ist, dass er sich im angelegten beziehungsweise angezogenen Zustand von oben auf dem Außenknöchel abstützt und der zweite Abstützbereich derart ausgebildet ist, dass er sich im angelegten beziehungsweise angezogenen Zustand von oben auf dem Innenknöchel abstützt, kann ein besonders gleichmäßiges Abstützen erzielt werden.

In einer bevorzugten weiteren Ausführungsform ist die Stützanbindung zumindest teilweise an einer ersten Seite der Stützanordnung, welche bevorzugt in einem an den Unterschenkel angebrachten Zustand einer lateralen Seite des Sprunggelenks entspricht, und zumindest teilweise an einer zweiten Seite der Stützanordnung, welche bevorzugt in einem an den Unterschenkel angebrachten Zustand einer medialen Seite des Sprunggelenks entspricht, mit der Stützanordnung verbunden. Dadurch erfolgt bei einer von der Halteeinrichtung kommenden Krafteinwirkung, beispielsweise infolge eines Umknickens, ein Verteilen der Kraft auf die erste Seite der Stützanordnung und die zweite Seite der Stützanordnung. Dabei jeweils in Umfangsrichtung des Unterschenkels beziehungsweise der Stützanordnung gerichtete Kraftkomponenten werden so in entgegengesetzte Richtungen wirkend eingeleitet. Dadurch wird die Gefahr eines Verrutschens aufgrund einer in Umfangsrichtung einseitig eingeleiteten beziehungsweise wirkenden Kraft verringert oder gar gänzlich vermieden.

Wenn die Stützanbindung gemäß einer weiteren bevorzugten Ausführungsform einen ersten Stützarm zum Anbinden an die Stützanordnung an der ersten Seite aufweist, und einen zweiten Stützarm zum Anbinden an die Stützanordnung an der zweiten Seite aufweist, kann die Stützanbindung besonders materialsparend ausgeführt werden. Ferner kann entsprechend der Anordnung der Stützarme eine durch ein Umknicken induzierte Kraft gezielt umgeleitet beziehungsweise in einer bevorzugten Richtung wirkend in die Stützanordnung eingeleitet werden. Zudem kann dadurch der Anatomie des Fußes beziehungsweise des Unterschenkels Rechnung getragen und Scheuerstellen an exponierten Stellen vermieden werden.

In einer besonders einfachen und robusten Ausführungsform sind der erste Stützarm und der zweite Stützarm einstückig ausgebildet.

Gemäß einer weiteren bevorzugten Ausführungsform ist ein Dämpfungselement in den ersten Stützarm integriert und/oder ein Dämpfungselement in den zweiten Stützarm integriert. Dadurch kann die Fußbewegungsdämpfungsvorrichtung besonders einfach und kompakt aufgebaut sein. Sind die Stützarme in einem Winkel zur Inversionsrichtung beziehungsweise Eversionsrichtung des Sprunggelenks angeordnet, verlängert sich ein Auszugsweg des jeweiligen Dämpfungselements entsprechend des zwischen dem jeweiligen Stützarm und der Inversionsrichtung beziehungsweise Eversionsrichtung eingeschlossenen Winkels. Durch das Vorliegen entsprechend größerer Auszugswege des Dämpfungselements kann das Dämpfungsverhalten des Dämpfungselements genauer eingestellt werden. Zudem hat ein in einen Stützarm integriertes Dämpfungselement lediglich die Kräfte beziehungsweise Kraftkomponenten aufzunehmen beziehungsweise zu dämpfen, die durch den jeweiligen Stützarm übertragen werden. Das Dämpfungselement kann entsprechend kleiner und einfacher aufgebaut sein.

In einer weiteren bevorzugten Ausführungsform ist ein Dämpfungselement in die Halteanbindung integriert. Dadurch kann eine Wirkrichtung des Dämpfungselements genau auf eine zu erwartende Inversionsrichtung angepasst beziehungsweise gemäß dieser vorgegeben werden.

Eine besonders effektive Dämpfung beziehungsweise Rückhaltung der Bewegung über das Sprunggelenk kann erzielt werden, wenn die Halteanbindung mit einer durch eine Sohle beziehungsweise Einlage der Halteanordnung definierten Ebene einen Winkel von 0° bis 90°, bevorzugt von 30° bis 70°, besonders bevorzugt von 50° bis 60° und ganz besonders bevorzugt 57° einschließt.

Unter "Sohle" wird hier ein Bereich verstanden, der im angezogenen Zustand der Vorrichtung flächig mit zumindest einem Teil der Fußunterseite des Trägers in Kontakt steht. Bevorzugt ist die Sohle als Einlage ausgebildet.

In einer bevorzugten weiteren Ausführungsform ist die Vorrichtung als komplett separate Vorrichtung bereitgestellt. Mit anderen Worten funktioniert die Vorrichtung ohne mit anderen Einrichtungen kombiniert werden zu müssen. Insbesondere ist es nicht notwendig, Teile der Vorrichtung an einen Schuh zu befestigen oder in diesen zu integrieren.

Um eine besonders wirkungsvolle Dämpfung der Sprunggelenkbewegung durch die Fußbewegungsdämpfungsvorrichtung erzielen zu können, ist gemäß einer weiteren bevorzugten Ausführungsform die Halteanbindung oder das in die Halteanbindung integrierte Dämpfungselement derart angeordnet, dass in einer Seitenansicht der Fußbewegungsdämpfungsvorrichtung, das heißt einer medialen Ansicht beziehungsweise lateralen Ansicht eines Fußes einer die Fußbewegungsdämpfungsvorrichtung tragenden Person, gesehen die resultierende Wirkrichtung beziehungsweise resultierende Wirklinie des Dämpfungselements die Sohle des Haltebereichs in etwa in einem Bereich des Grundgelenks D5 des Fußes, das heißt im Bereich zwischen dem Mittelfußknochen und dem Grundglied des kleinen Zehs des Fußes, schneidet.

Damit die Beweglichkeit des die Fußbewegungsdämpfungsvorrichtung tragenden Fußes nur eine geringe Beeinflussung erfährt, ist gemäß einer weiteren bevorzugten Ausführungsform ein Übergang zwischen der Stützanbindung und der Halteanbindung gelenkig beziehungsweise beweglich ausgebildet. Bevorzugt ist der Übergang in Form eines Schubgelenks, eines Drehgelenks oder eines Querkraftgelenks ausgebildet. Alternativ kann der Übergang als Umlenkung ausgebildet sein, wobei der Übergang dann bevorzugt eine Rolle, eine Öse und/oder eine Schlaufe aufweist. Beispielsweise bei einer Plantarflexion oder Dorsalextension des die Fußbewegungsdämpfungsvorrichtung tragenden Fußes kann so ein Abrollen beziehungsweise Verschieben der Stützanbindung relativ zur Halteanbindung im Bereich des Übergangs erfolgen, ohne dass der Umfang der vorgenannten Bewegung durch die Fußbewegungsdämpfungsvorrichtung eingeschränkt wird.

In einer weiteren bevorzugten Ausführungsform ist die Fußbewegungsdämpfungsvorrichtung an einem Socken, einem Stutzen, einer Orthese oder einer Bandage angeordnet.

In einer weiteren bevorzugten Ausführungsform weist zumindest einer der Stützarme einen einstellbaren Anbindungsbereich zum Anpassen einer Länge der Stützanbindung auf. Bevorzugt ist die einstellbare Anordnung in Form einer Rastanordnung, einer Hakenverbindung oder einer Klettverbindung ausgebildet. Bevorzugt wird der einstellbare Anbindungsbereich durch das Anlegen der Abstützfixierung fixiert.

Die oben genannte Aufgabe wird ferner durch einen Schuh zum Dämpfen einer Fußbewegung über das Sprunggelenk mit den Merkmalen des Anspruchs 10 gelöst. Vorteilhafte Weiterbildungen ergeben sich aus den Unteransprüchen, der Beschreibung und den Figuren.

Entsprechend wird ein Schuh zum Dämpfen einer Fußbewegung über das Sprunggelenk vorgeschlagen, welcher eine Sohle und ein Oberteil umfasst. Erfindungsgemäß ist an dem Schuh eine Fußbewegungsdämpfungsvorrichtung gemäß einem der vorstehenden Ausführungsformen angeordnet. Dadurch, dass an dem Schuh eine Fußbewegungsdämpfungsvorrichtung gemäß einem der vorstehenden Ausführungsformen vorgesehen ist, kann ein Anwender beziehungsweise Träger des Schuhs die vorstehend beschriebenen Vorteile nutzen.

Als "Schuh" wird hier jede Form von schuhartiger Fußbekleidung mit einem Oberteil beziehungsweise Schuhschaft und einer damit verbundenen festen Unterlage beziehungsweise Sohle verstanden, insbesondere orthopädische Schuhe, Sportschuhe, Freizeitschuhe und Stiefel.

In einer weiteren bevorzugten Ausführungsform des Schuhs ist die Dämpfungsanordnung zumindest teilweise an einer Außenseite des Schuhs angeordnet. Dadurch sind die bei einem Umknicken resultierenden Wege, insbesondere der resultierende Auszugsweg größtmöglich, was ein besonders genaues Einstellen der Dämpfungsanordnung und ein besonders effektives Dämpfen ermöglicht. Ferner ist ein einfaches Nachrüsten eines Schuhs mit der Fußbewegungsdämpfungsvorrichtung sowie ein einfaches Warten und/oder Austauschen von Teilen möglich.

In einer weiteren bevorzugten Ausführungsform des Schuhs ist die Halteanbindung zumindest teilweise in den Schuh integriert und/oder die Stützanbindung zumindest teilweise in den Schuh integriert und/oder zumindest ein Dämpfungselement in den Schuh integriert, wobei bevorzugt die gesamte Dämpfungsanordnung in den Schuh integriert ist. Dadurch kann zum einen ein besonders steifer und kompakter Aufbau des Schuhs erzielt werden. Weiterhin kann der Schuh dann so ausgebildet sein, dass auf der Außenseite des Schuhs weniger beziehungsweise keine Teile hervorstehen, die bei einem Gebrauch des Schuhs, beispielsweise beim Fußballspielen, die Ausübung des Sports beeinträchtigen. Zudem kann der Schuh derart ausgebildet sein, dass er sich von einem Schuh, welcher keine Fußbewegungsdämpfungsvorrichtung zum Dämpfen aufweist, von außen betrachtet nicht oder nur unwesentlich unterscheidet. Somit kann das ästhetische Erscheinungsbild des Schuhs gegenüber dem eines herkömmlichen Schuhs weitestgehend gewahrt werden.

In einer weiteren bevorzugten Ausführungsform des Schuhs ist die Stützanordnung als separate Komponente bereitgestellt. Unter "separat bereitgestellt" ist hierbei zu verstehen, dass die Stützanordnung nur über die Stützanbindung beziehungsweise die Dämpfungsanordnung mit dem Schuh verbunden ist. Dadurch kann der Schuh im Wesentlichen entsprechend einem Schuh, welcher keine Fußbewegungsdämpfungsvorrichtung zum Dämpfen aufweist, ausgebildet sein. Die separate Stützanordnung ist dann eine nicht mit dem Textil des Schuhes verbundene Komponente. Die einzige Verbindung zwischen dem eigentlichen Schuh und der Stützanordnung ist entsprechend die Stützanbindung. Insbesondere bei Schuhen mit niedrigem Schaft, wie Laufschuhe oder Fußballschuhe, kann so eine maximale Beweglichkeit des Fußes erzielt werden.

In einer besonders bevorzugten, weiteren Ausführungsform weist der Schuh einen hohen Schaft auf, wobei die Stützanordnung in den hohen Schaft integriert ist. Dadurch ist ein separates Vorsehen der Stützanordnung nicht notwendig. Zudem ist ein Verschieben der Stützanordnung dadurch im Wesentlichen verhindert. Bevorzugt ist die Abstützfixierung als Schlaufe, Band beziehungsweise Schlinge mit einer Öse an der Außenseite des Schuhes angeordnet. Alternativ kann die Abstützfixierung auch über zugsteife Bereiche des Schaftes und einer primären Schließeinrichtung des Schuhs, beispielsweise einer Schnürung eines Schnürschuhs oder eines Klettverschlusses eines Klettverschlussschuhs, ausgebildet sein.

### Kurze Beschreibung der Figuren

Bevorzugte weitere Ausführungsformen der Erfindung werden durch die nachfolgende Beschreibung der Figuren näher erläutert. Dabei zeigen:
- Figur 1: schematisch eine perspektivische Seitenansicht einer Fußbewegungsdämpfungsvorrichtung zum Dämpfen einer Fußbewegung über das Sprunggelenk gemäß einer ersten Ausführungsform;
- Figur 2: schematisch eine Seitenansicht einer Fußbewegungsdämpfungsvorrichtung zum Dämpfen einer Fußbewegung über das Sprunggelenk gemäß einer weiteren Ausführungsform;
- Figur 3: schematisch eine Seitenansicht eines niederschaftigen Schuhs zum Dämpfen einer Fußbewegung über das Sprunggelenk gemäß einer ersten Ausführungsform;
- Figur 4: schematisch eine Seitenansicht eines hochschaftigen Schuhs zum Dämpfen einer Fußbewegung über das Sprunggelenk gemäß einer weiteren Ausführungsform;
- Figur 5: schematisch den Schuh aus Figur 4 von einer anderen Seite gesehen;
- Figur 6: schematisch eine Seitenansicht eines niederschaftigen Schuhs zum Dämpfen einer Fußbewegung über das Sprunggelenk gemäß einer weiteren Ausführungsform;
- Figur 7: schematisch eine Seitenansicht eines hochschaftigen Schuhs zum Dämpfen einer Fußbewegung über das Sprunggelenk gemäß einer weiteren Ausführungsform; und
- Figur 8: schematisch den Schuh aus Figur 7 von einer anderen Seite gesehen.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen versehen. Um Redundanzen zu vermeiden, wird auf eine wiederholte Beschreibung dieser Elemente teilweise verzichtet.

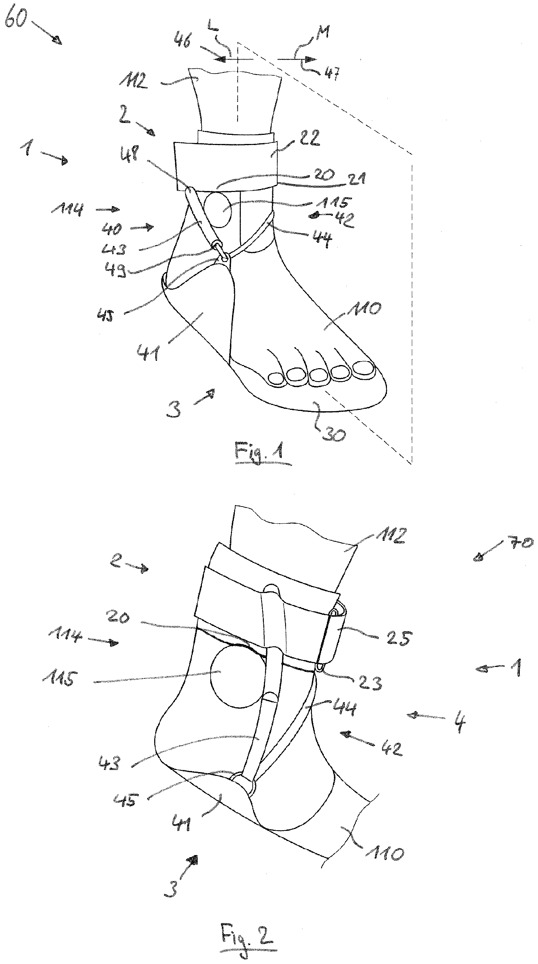

In Figur 1 ist schematisch eine perspektivische Seitenansicht einer Fußbewegungsdämpfungsvorrichtung 1 zum Dämpfen einer Fußbewegung über das Sprunggelenk gemäß einer ersten Ausführungsform, welche an einem menschlichen Fuß 110 angebracht ist, gezeigt. Die Fußbewegungsdämpfungsvorrichtung 1 ist in Form einer Orthese 60 ausgebildet. Die Fußbewegungsdämpfungsvorrichtung 1 ist unterhalb des Knöchels 114, der Malleolengabel, des Sprunggelenks des Fußes 110 über eine Halteanordnung 3 an den Fuß 110 angebunden. Oberhalb des Knöchels 114 ist die Fußbewegungsdämpfungsvorrichtung 1 mittels einer Stützanordnung 2 an einen Unterschenkel 112 angebunden. Die Stützanordnung 2 und die Halteanordnung 3 sind über eine Dämpfungsanordnung 4 verbunden, welche eine Relativbewegung zwischen der Stützanordnung 2 und der Halteanordnung 3 adaptiv, vorliegend geschwindigkeitsabhängig und beschleunigungsabhängig, dämpft. Die Fußbewegungsdämpfungsvorrichtung 1 gemäß Figur 1 ist dabei derart ausgebildet, dass sie eine Inversionsbewegung des Sprunggelenks adaptiv dämpfen kann.

Zur Anbindung an den Fuß 110 weist die Halteanordnung 3 eine Sohle 30 auf, auf welcher die Fußsohle des Fußes 110 aufliegt. Die Sohle 30 ist an einer ersten Seite 46 der Fußbewegungsdämpfungsvorrichtung 1, welche hier der lateralen Seite L des Fußes 110 entspricht, mit einer Halteanbindung 41 der Dämpfungsanordnung 4 verbunden. Die Halteanbindung 41 ist mittels eines Übergangs 45, vorliegend in Form einer an der Halteanbindung 41 angebrachten Öse mit einer Stützanbindung 42 der Dämpfungsanordnung 4 verbunden. Über die Stützanbindung 42 wiederum ist die Dämpfungsanordnung 4 mit der Stützanordnung 2 verbunden. Die Stützanbindung 42 ist einteilig in Form eines zugsteifen Bandes ausgebildet, dass in der Öse des Übergangs 45 umgelenkt wird. Dadurch ist die Stützanbindung 42 in einen ersten und einen zweiten Stützarm 43, 44 unterteilt. Der erste Stützarm 43 erstreckt sich auf der ersten Seite 46 der Fußbewegungsdämpfungsvorrichtung 1 beziehungsweise der Stützanordnung 2 zwischen dem Übergang 45 und der Stützanordnung 2. Der zweite Stützarm 44 erstreckt sich von dem auf der ersten Seite 46 gelegenen Übergang 45 auf die zweite Seite 47 der Fußbewegungsdämpfungsvorrichtung 1 beziehungsweise der Stützanordnung 2 und ist dort mit der Stützanordnung 2 verbunden. Die Stützanbindung 42 beziehungsweise das Band ist beweglich in dem Übergang 45 geführt. Bei einer Plantarflexion oder einer Dorsalextension über das Sprunggelenk (bzw. über dessen obere Sprunggelenksachse) verschiebt sich der Abschnitt, an dem das Band der Stützanbindung 42 in der Öse umgelenkt wird entsprechend des Umfangs der Bewegung. Dadurch ändern sich die Längen des ersten Stützarms 43 und des zweiten Stützarms 44 geringfügig. Diese geringfügige Änderung wird durch ein geringfügiges Gleiten des Bandes in der Öse ausgeglichen, so dass bei einer festen Anbindung etwaige entstehende Falten oder Druckstellen auf der Haut vermieden werden.

Erfährt das Sprunggelenk eine Inversion, vergrößert sich auf der ersten Seite 46 der Abstand zwischen Halteanordnung 3 und Stützanordnung 2. Mithin vergrößert sich der Abstand zwischen der Stützanordnung 4 und dem Übergang 45. Dementsprechend müsste sich auch die Länge der Stützanbindung 42 von einem ersten Anbindungsbereich an der Stützanordnung 2 auf der ersten Seite 46 über die Öse des Übergangs 45 und zu einem zweiten Anbindungsbereich an der Stützanordnung 2 auf der zweiten Seite 47 verlängern. Um solch eine Bewegung im physiologischen Bereich grundsätzlich zu ermöglichen, weist die Dämpfungsanordnung 4 ein Dämpfungselement 40 auf, welches in den ersten Stützarm 43 integriert ist. Das Dämpfungselement 4 weist einen rohrförmigen ersten Dämpferteil 48 auf, der fest mit der Stützanordnung 2 verbunden ist, und weist einen relativ zum ersten Dämpferteil 48 entlang einer Auszugsrichtung, welche sich entlang der Längsachse des rohrförmigen ersten Dämpferteils 48 erstreckt, beweglichen, zweiten Dämpferteil 49 auf, der mit dem Band der Stützanbindung 42 verbunden ist. Der zweite Dämpferteil 49 erstreckt sich teilweise im Inneren des rohrförmigen ersten Dämpferteils 48 und weist darin einen Auszugskörper aus. In dem Dämpfungselement ist ferner ein Dämpfungsmedium enthalten. Bei einer Relativbewegung des zweiten Dämpferteils 49 gegenüber dem ersten Dämpferteil 48 wird die Bewegung des zweiten Dämpferteils 49 entsprechend der Geometrie der Innenseite des ersten Dämpferteils 48 und des Auszugskörpers des zweiten Dämpferteils 49 sowie der Beschaffenheit, insbesondere der Viskosität, des Dämpfungsmediums gedämpft. Das Dämpfungselement 40 ist dabei derart ausgestaltet, dass es die Bewegungen adaptiv dämpft. Bei Bewegungen mit moderaten Geschwindigkeiten beziehungsweise Beschleunigungen kann so eine nahezu ungehinderte Beweglichkeit des Sprunggelenks bereitgestellt werden. Tritt eine Inversion mit hohen Geschwindigkeiten und/oder Beschleunigungen auf, bei welchen die Gefahr von Verletzungen des Bandapparats des Sprunggelenks besteht, dämpft das Dämpfungselement 40 die Inversionsbewegung und eine Verletzung wird vermieden.

Das Dämpfungselement 40 weist ferner eine Dichtung zum Abdichten des Innenraums des das Dämpfungsmedium aufweisenden ersten Dämpferteils 48 und eine Rückstellung zum Rückstellen des zweiten Dämpferteils 49 in eine in den ersten Dämpferteil 48 eingefahrene beziehungsweise eingeschobene Position auf. Durch die Rückstellung ist sichergestellt, dass der zweite Dämpferteil 49 wieder in den ersten Dämpferteil 48 bewegt wird, wenn der Fuß aus einer Inversionsstellung zurück bewegt wird. Dadurch erfolgt die Schutzwirkung der Fußbewegungsdämpfungsvorrichtung 1 nicht erst bei beziehungsweise nach Erreichen eines bestimmten Grenzinverisonswinkels, sondern steht bei im Wesentlichen jeder Stellung des Fußes zur Verfügung.

Damit sich die Stützanordnung 2 bei einer Krafteinwirkung infolge einer Inversion nicht verschiebt, insbesondere nicht in Richtung des Fußes 110 nach unten rutscht, weist die Stützanordnung 2 einen ersten und einen zweiten Abstützbereich 20, 21 auf. Der erste Abstützbereich 20 ist an der ersten Seite 46 der Stützanordnung 2 so angeordnet, dass er sich, wenn die Fußbewegungsdämpfungsvorrichtung 1 am Fuß 110 beziehungsweise Unterschenkel 112 angebracht ist, im Wesentlichen oberhalb des Außenknöchels 115, des Malleolus lateralis, befindet. Gegenüberliegend, das heißt auf der zweiten Seite 47, weist die Stützanordnung 2 einen hier nicht gezeigten zweiten Abstützbereich 21 auf. Der zweite Abstützbereich 21 ist dabei so an der Stützanordnung 2 angeordnet, dass er sich, wenn die Fußbewegungsdämpfungsvorrichtung 1 am Fuß 110 beziehungsweise Unterschenkel 112 angebracht ist, im Wesentlichen oberhalb des Innenknöchels, des Malleolus medialis, befindet. Die Stützanordnung 2 stützt sich bei einer Inversion, bei welcher über die Stützanbindung 42 eine Zugkraft in Richtung des Fußes 110 in die Stützanordnung 2 eingeleitet wird, mit dem ersten Abstützbereich 20 von oben auf dem Außenknöchel 115 und mit dem zweiten Abstützbereich 21 von oben auf dem Innenknöchel des Sprunggelenks abstützt. Um zu verhindern, dass sich die Stützanordnung 2 radial aufweitet und so die Abstützbereiche 20, 21 über den Knöchel 114 nach unten Richtung Fuß 110 rutschen, weist die Stützanordnung 2 eine Abstützfixierung 22 auf, mittels welcher die Position des ersten Abstützbereichs 20 und des zweiten Abstützbereichs 21 an dem Unterschenkel fixiert wird. Die Abstützfixierung 22 ist dabei als Klettband 25 mit einer Umlenköse und einem Klettverschluss ausgebildet, so dass sie über die Umlenköse am Unterschenkel 112 straff gezogen und mittels des Klettverschlusses fixiert werden kann.

Die Fußbewegungsdämpfungsvorrichtung 1 aus Figur 1 ist als komplett separate Vorrichtung ausgebildet, welche ihre Schutzwirkung aufgrund ihres Aufbaus entfaltet. Es ist nicht erforderlich, dass eine die Vorrichtung 1 tragende Person zum Erzielen der vorgenannten Schutzwirkung über die Vorrichtung 1 einen Schuh ziehen muss. Die Vorrichtung 1 kann jedoch derart ausgebildet sein, dass ein Tragen der Vorrichtung 1 unter beziehungsweise in einem Schuh möglich ist, ohne dass es zu Druckstellen am Fuß kommt. Hierzu kann die Sohle 30 bevorzugt als Einlage ausgebildet sein, welche eine Einlage in einem Schuh ersetzt oder auf diese aufgelegt werden kann.

Figur 2 zeigt schematisch eine Seitenansicht von einer ersten Seite 46, der lateralen Seiten des Sprunggelenks, einer Fußbewegungsdämpfungsvorrichtung 1 zum Dämpfen einer Fußbewegung über das Sprunggelenk gemäß einer weiteren Ausführungsform, in welcher die Fußbewegungsdämpfungsvorrichtung 1 an einer Bandage 70 vorgesehen ist. Die Bandage 70 weist als Grundstruktur einen Strumpf 72 aus einem elastischen Kompressionsgestrick, das sich dem Körper des Trägers anpassen kann, auf.

An dem Strumpf 72 der Bandage 70 ist die Stützanordnung 2 derart angeordnet, dass sie, wenn die Bandage 70 an Fuß 110 und Unterschenkel 112 angebracht ist, sich oberhalb des Knöchels 114 befindet. Die Stützanordnung 2 weist entsprechend der Ausführungsform aus Figur 1 einen ersten Abstützbereich 20 zum Abstützen am Außenknöchel 115 und einen zweiten Abstützbereich 21 (nicht gezeigt) zum Abstützen auf den Innenknöchel auf. An der Unterseite der Bandage 70 weist diese eine zugsteife Halteanordnung 3 auf, welche seitlich mit einer als kleine Gewebelasche ausgebildeten Halteanbindung 41 verbunden ist. an der Halteanbindung 41 ist ein Übergang 45 in Form einer Schlaufe angeordnet. Über die Schlaufe wird die Stützanbindung 42, welche entsprechend der Ausführungsform der Fußbewegungsdämpfungsvorrichtung 1 aus Figur 1 als zugsteifes Band ausgebildet ist, von einem auf der ersten Seite 46 gelegenen ersten Anbindungsbereich 43a an der Stützanordnung 2 kommend umgelenkt in Richtung eines (nicht gezeigten) auf der zweiten Seite 47 gelegenen zweiten Anbindungsbereichs 44a. Mithin ist die Stützanbindung 42 wiederum in einen ersten Stützarm 43 zum Anbinden an die Stützanordnung 2 an der ersten Seite 46 und einen zweiten Stützarm 44 zum Anbinden an die Stützanordnung 2 an der zweiten Seite 47 unterteilt.

Das Dämpfungselement 40 ist hier im Anbindungsbereich 43a angeordnet und erstreckt sich im Wesentlichen über die gesamte Breite des Klettbands 25 der Stützanordnung 2. Dadurch weist die Bandage 70 unterhalb des Knöchels 114 keine starken Ausprägungen auf. Die Bandage 70 ist daher insbesondere dazu geeignet, sie unterhalb eines niederschaftigen Schuhs zu tragen, ohne dass am Fuß Druckstellen durch etwaige Ausprägungen entstehen.

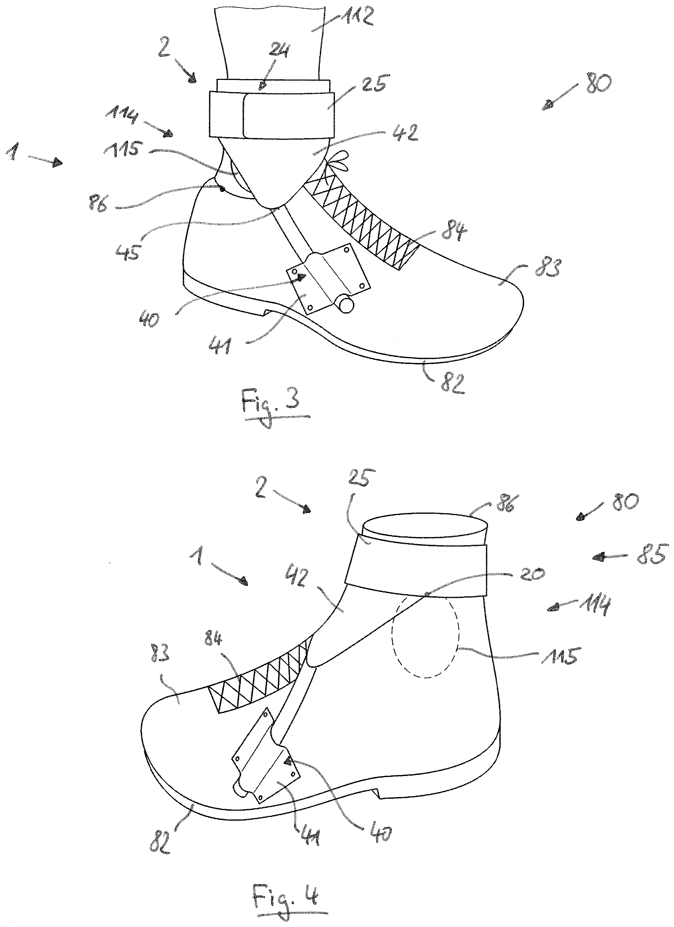

Figur 3 zeigt schematisch eine Seitenansicht von der ersten Seite 46 eines niederschaftigen Schuhs 80 zum Dämpfen einer Fußbewegung über das Sprunggelenk gemäß einer ersten Ausführungsform. Am dem Schuh 80 ist eine Fußbewegungsdämpfungsvorrichtung 1 zum Dämpfen einer Fußbewegung über das Sprunggelenk vorgesehen. Der Schuh 80 ist als Sport-Schnürschuh mit einer Schuhsohle 82 und einem Oberteil, dem Schuhschaft 83, dessen Schaftabschluss 86 im angezogenen Zustand unterhalb des Knöchels 114 angeordnet ist, ausgebildet. Der Schuhschaft 83 erstreckt sich nicht über den Knöchel 114.

Die Halteanordnung 3 wird durch die Schuhsohle 82 und das Oberteil, das heißt den Schuhschaft 83, ausgebildet. Die positionsstabile Anbindung an den Fuß 110 wird durch die Schnürung 84 erzielt, mittels derer der Schuhschaft 83 an die Kontur des Fußes 110 gezogen werden kann. Auf der ersten Seite 46, das heißt der lateralen Seite des Fußes 110, ist an dem Schuhschaft 83 eine Halteanbindung 41 in Form eines formstabilen Flansches befestigt. An dem Flansch ist ein Dämpfungselement 40 befestigt. Das Dämpfungselement 40 ist am Übergang 45 mit einer Stützanbindung 42 in Form eines zugsteifen Gewirks fest verbunden. Die Stützanbindung 42 ist mit einem Teil an der ersten Seite 46 der Stützanordnung 2, welche im an den Unterschenkel 112 angebrachten Zustand einer lateralen Seite des Sprunggelenks entspricht, und mit einem weiteren Teil an einer zweiten Seite 47 der Stützanordnung 2, welche im an den Unterschenkel 112 angebrachten Zustand einer medialen Seite des Sprunggelenks entspricht, mit der Stützanordnung 2 verbunden. Von der Dämpfungsanordnung 4 über die Stützanbindung 41 in die Stützanordnung 2 eingeleitet Kräfte werden somit sowohl auf der ersten Seite 46 als auch auf der zweiten Seite 47 in die Stützanordnung 2 eingeleitet.

Die Stützanordnung 2 ist in der in Figur 4 gezeigten Ausführungsform als separate Komponente ausgebildet. Mithin ist die Stützanordnung 2 nur über die Stützanbindung 42 der Dämpfungsanordnung 4 mit dem Schuhschaft 83 verbunden. Im angezogenen Zustand besteht somit ein Abstand zwischen der oberhalb des Knöchels 114 angeordneten Stützanordnung 2 und dem unterhalb des Knöchels 114 gelegenen Schaftabschluss 86.

Figur 4 ist schematisch eine Seitenansicht eines hochschaftigen Schuhs 80 mit einer Fußbewegungsdämpfungsvorrichtung 1 zum Dämpfen einer Fußbewegung über das Sprunggelenk gezeigt. Der grundsätzliche Aufbau des Schuhs 80 entspricht im Wesentlichen dem des Schuhs aus Figur 3, er unterscheidet sich jedoch dadurch, dass der Schuh 80 aus Figur 4 einen hohen Schaft 85 aufweist. Somit liegt der Schaftabschluss 86 des Schuhs 80 im angezogenen Zustand oberhalb des hier mit dem Bezugszeichen 114 angedeuteten Knöchels 114. Ferner ist die Stützanordnung 2 in den Schuh 80 integriert. Die Stützanordnung 2 weist einen ersten Abstützbereich 20 zum Abstützen von oben auf dem Außenknöchel 115 auf der ersten Seite 46 und einen zweiten (hier verdeckten) Abstützbereich 21 zum Abstützen von oben auf dem (nicht gezeigten) Innenknöchel 116 auf der zweiten Seite 47 auf. Die Abstützbereiche 20, 21 sind im Inneren des hohen Schaftes 85 angeordnet. Um die Stabilität der Stützanordnung 2 zu gewährleisten, ist an dem hohen Schaft 85 eine Abstützfixierung 22 in Form eines Klettbandes 25 vorgesehen.

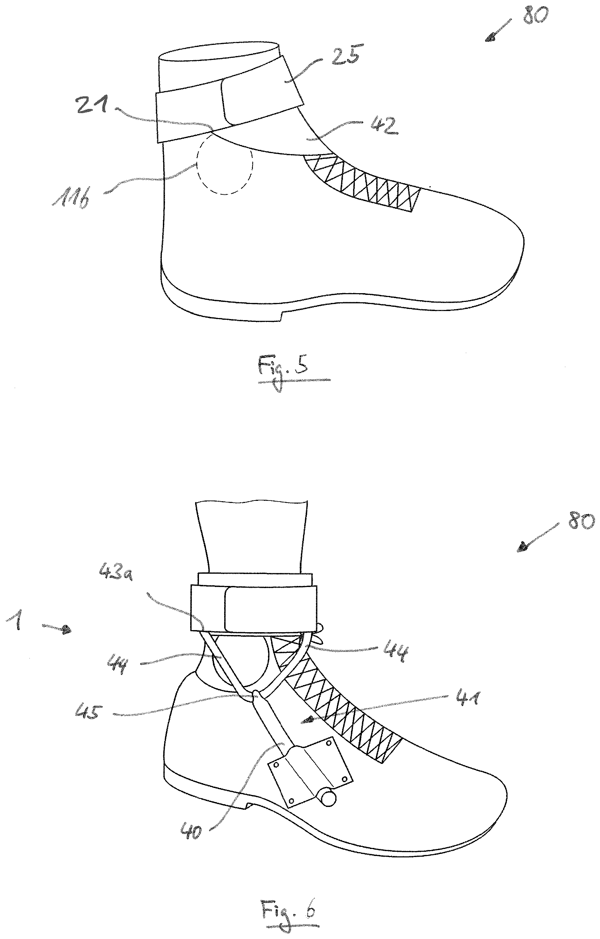

Figur 5 zeigt schematisch den Schuh 80 aus Figur 4 von der zweiten Seite 47, der medialen Seite des Sprunggelenks, aus gesehen. Die Stützanbindung 42 erstreckt sich auf der zweiten Seite 47 in etwa bis zum hinteren Ende der hier mit dem Bezugszeichen 116 angedeuteten Lage des Innenknöchels.

Figur 6 zeigt schematisch eine Seitenansicht eines niederschaftigen Schuhs 80 mit einer Fußbewegungsdämpfungsvorrichtung 1 zum Dämpfen einer Fußbewegung über das Sprunggelenk gemäß einer weiteren Ausführungsform. Der Schuh 80 entspricht weitestgehend dem aus Figur 3. Einziger Unterschied ist, dass der in Figur 6 gezeigte Schuh 80 anstatt des flächigen Gewirks eine Stützanbindung 42 in Form eines zugsteifen Bandes aufweist, das durch den Übergang 45 zur Halteanbindung 41 in einen ersten Stützarm 43 und einen zweien Stützarm 44 unterteilt ist. Der erste Stützarm 43 ist an einem auf der ersten Seite 46 gelegenen ersten Anbindungsbereich 43a mit der Stützanordnung 2 verbunden und ist an einem auf der zweiten Seite 47 gelegenen (nicht gezeigten) zweiten Anbindungsbereich 44a mit der Stützanordnung 2 verbunden. Der Übergang 45 ist als feste Naht ausgebildet.

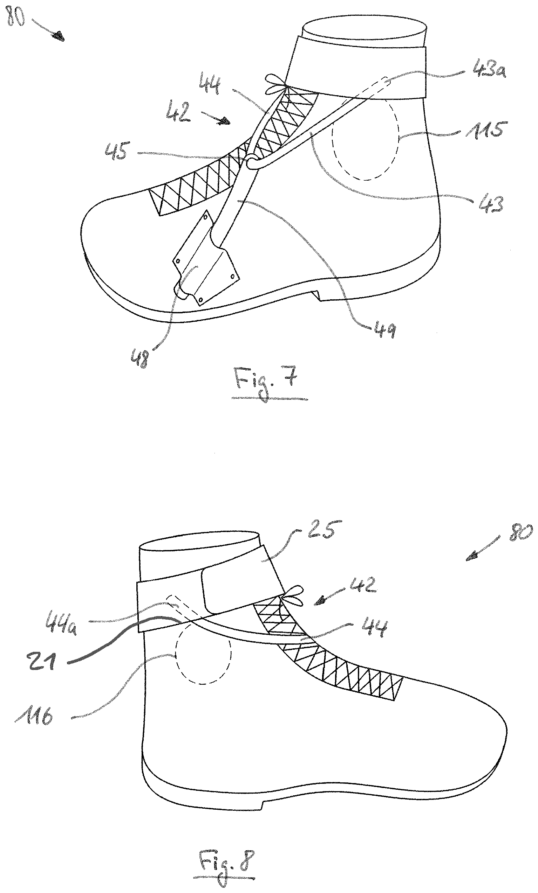

In Figur 7 ist schematisch eine Seitenansicht eines hochschaftigen Schuhs 80 mit einer Fußbewegungsdämpfungsvorrichtung 1 zum Dämpfen einer Fußbewegung über das Sprunggelenk gemäß einer weiteren Ausführungsform gezeigt. Der hochschaftige Schuh 80 entspricht jenem aus Figur 4, wobei anstatt des flächigen Gewirks die Stützanbindung 42 in Form einer zugsteifen Schnur mit im Wesentlichen rundem Querschnitt bereitgestellt ist, welches durch den Übergang 45 zur Halteanbindung 41 in einen ersten Stützarm 43 und einen zweien Stützarm 44 unterteilt ist. Der erste Stützarm 43 ist an einem auf der ersten Seite 46 gelegenen ersten Anbindungsbereich 43a mit der Stützanordnung 2 verbunden und ist an einem auf der zweiten Seite 47 gelegenen (nicht gezeigten) zweiten Anbindungsbereich 44a mit der Stützanordnung 2 verbunden. Der Übergang 45 ist als Öse am Ende des zweiten Dämpferteils 49 ausgebildet, in welcher die Schnur beweglich gehalten ist.

Figur 8 zeigt schematisch den Schuh 80 aus Figur 7 von der zweiten Seite 47, der medialen Seite des Sprunggelenks, aus gesehen. Die Stützanbindung 42 erstreckt sich auf der zweiten Seite 47 in etwa bis zum hinteren Ende der hier mit dem Bezugszeichen 116 angedeuteten Lage des Innenknöchels. Ferner ist mit dem Bezugszeichen 44a der unterhalb des Klettbands angeordnete zweite Anbindungsbereich angedeutet.

Anstatt einer starren Anbindung kann bei den vorgenannten Ausführungsformen der erste Anbindungsbereich 43a und/oder der zweite Anbindungsbereich 44a einstellbar ausgebildet sein. Beispielsweise kann zumindest einer der Stützarme 43, 44 eine Rastanordnung, eine Hakenverbindung mit mehreren an der Stützanbindung in Reihe angeordneten Haken oder eine Klettverbindung aufweisen. Bevorzugt wird der zumindest eine einstellbare Anbindungsbereich 43a, 44a durch das Anlegen der Abstützfixierung 22 zusätzlich fixiert.

Soweit anwendbar, können alle einzelnen Merkmale, die in den Ausführungsbeispielen dargestellt sind, miteinander kombiniert und/oder ausgetauscht werden, ohne den Bereich der Erfindung zu verlassen, wobei der Schutzbereich der Erfindung in den Ansprüchen definiert ist.

### Bezuqszeichenliste

- 1: Fußbewegungsdämpfungsvorrichtung
- 2: Stützanordnung
- 20: Abstützbereich
- 22: Abstützfixierung
- 23: Umlenköse
- 24: Klettverschluss
- 25: Klettband
- 3: Halteanordnung
- 30: Sohle
- 4: Dämpfungsanordnung
- 40: Dämpfungselement
- 41: Halteanbindung
- 42: Stützanbindung
- 43: Erster Stützarm
- 43a: Erster Anbindungsbereich
- 44: Zweiter Stützarm
- 44a: Zweiter Anbindungsbereich
- 45: Übergang
- 46: Erste Seite
- 47: Zweite Seite
- 48: Erster Dämpferteil
- 49: Zweiter Dämpferteil

- 60: Orthese
- 70: Bandage
- 72: Strumpf
- 80: Schuh
- 82: Schuhsohle
- 83: Schuhschaft
- 84: Schnürung
- 85: Hoher Schaft
- 86: Schaftabschluss
- 110: Fuß
- 112: Unterschenkel
- 114: Knöchel
- 115: Außenknöchel
- 116: Innenknöchel
- M: Mediale Seite
- L: Laterale Seite

## Patentansprüche

1. Fußbewegungsdämpfungsvorrichtung (1) zum Dämpfen einer Fußbewegung über das Sprunggelenk,
umfassend eine Stützanordnung (2) zum Abstützen an einem Unterschenkel (112) und eine Halteanordnung (3) zum Halten an einem Fuß (110),
wobei eine Dämpfungsanordnung (4) mit mindestens einem Dämpfungselement (40) zum Dämpfen einer Relativbewegung zwischen der Stützanordnung (2) und der Halteanordnung (3) angeordnet ist,
**dadurch gekennzeichnet, dass**
die Dämpfungsanordnung (4) eine Stützanbindung (42) zum Anbinden an die Stützanordnung (2) und eine Halteanbindung (41) zum Anbinden an die Halteanordnung (3) aufweist, wobei die Stützanbindung (42) zumindest teilweise an einer ersten Seite (46) der Stützanordnung (2), welche in einem an den Unterschenkel (112) angebrachten Zustand einer lateralen Seite (L) des Sprunggelenks entspricht, und zumindest teilweise an einer zweiten Seite (47) der Stützanordnung (2), welche in einem an den Unterschenkel (112) angebrachten Zustand einer medialen Seite (M) des Sprunggelenks entspricht, mit der Stützanordnung (2) verbunden ist,
wobei die Stützanbindung (42) einen ersten Stützarm (43) zum Anbinden an die Stützanordnung (2) an der ersten Seite (46) aufweist, und einen zweiten Stützarm (44) zum Anbinden an die Stützanordnung (2) an der zweiten Seite (47) aufweist,
wobei das Dämpfungselement (40) in den ersten Stützarm (43) integriert ist und/oder ein/das Dämpfungselement (40) in den zweiten Stützarm (44) integriert ist;
**und dadurch, dass** die Stützanordnung (2) zumindest einen Abstützbereich (20, 21) und mindestens eine Abstützfixierung (22) umfasst, wobei sich die Stützanordnung (2) mit dem zumindest einen Abstützbereich (20, 21) oberhalb des Knöchels (114) des Sprunggelenks an dem Knöchel (114) abstützen kann, und wobei die mindestens eine Abstützfixierung (22) zum Fixieren der Position des zumindest einen Abstützbereichs (20, 21) in einem an den Unterschenkel (112) angebrachten Zustand ausgebildet ist.

2. Fußbewegungsdämpfungsvorrichtung (1) gemäß Anspruch 1, **dadurch gekennzeichnet, dass** die Stützanordnung (2) zumindest einen ersten Abstützbereich (20) zum Abstützen an einer ersten Seite (46), bevorzugt einer lateralen Seite (L) des Sprunggelenks, und zumindest einen zweiten Abstützbereich (21) zum Abstützen an einer zweiten Seite (47), bevorzugt einer medialen Seite (M) des Sprunggelenks, aufweist.

3. Fußbewegungsdämpfungsvorrichtung (1) gemäß Anspruch 1 oder 2, **dadurch gekennzeichnet, dass** der erste Stützarm (43) und der zweite Stützarm (44) einstückig ausgebildet sind.

4. Fußbewegungsdämpfungsvorrichtung (1) gemäß einem der vorstehenden Ansprüche, **dadurch gekennzeichnet, dass** ein Dämpfungselement (40) in die Halteanbindung (41) integriert ist.

5. Fußbewegungsdämpfungsvorrichtung (1) gemäß Anspruch 4, **dadurch gekennzeichnet, dass** die Halteanbindung (41) mit einer durch eine Sohle (30) der Halteanordnung (3) definierten Ebene einen Winkel von 0° bis 90°, bevorzugt von 30° bis 70°, besonders bevorzugt von 50° bis 60° und ganz besonders bevorzugt 57° einschließt.

6. Fußbewegungsdämpfungsvorrichtung (1) gemäß einem der vorstehenden Ansprüche, **dadurch gekennzeichnet, dass** ein Übergang (45) zwischen der Stützanbindung (42) und der Halteanbindung (41) gelenkig beziehungsweise beweglich ausgebildet ist.

7. Schuh (80) zum Dämpfen einer Fußbewegung über das Sprunggelenk, umfassend eine Sohle (82) und ein Oberteil, **dadurch gekennzeichnet, dass** der Schuh (80) eine Fußbewegungsdämpfungsvorrichtung (1) gemäß einem der vorstehenden Ansprüche aufweist.

8. Schuh (80) gemäß Anspruch 7, **dadurch gekennzeichnet, dass** die Dämpfungsanordnung (4) zumindest teilweise an einer Außenseite des Schuhs (80) angeordnet ist.

9. Schuh (80) gemäß Anspruch 7 oder 8, **dadurch gekennzeichnet, dass** die Halteanbindung (41) zumindest teilweise in den Schuh (80) integriert ist und/oder die Stützanbindung (42) zumindest teilweise in den Schuh (80) integriert ist und/oder zumindest ein Dämpfungselement (40) in den Schuh (80) integriert ist, wobei bevorzugt die gesamte Dämpfungsanordnung (4) in den Schuh (80) integriert ist.

10. Schuh (80) gemäß einem der Ansprüche 7 bis 9, **dadurch gekennzeichnet, dass** die Stützanordnung (2) als separate Komponente bereitgestellt ist.

11. Schuh (80) gemäß einem der Ansprüche 7 bis 9, **dadurch gekennzeichnet, dass** der Schuh (80) einen hohen Schaft (85) aufweist, wobei die Stützanordnung (2) in den hohen Schaft (85) integriert ist.

## Claims

1. A foot movement damping device (1) for damping a foot movement via the ankle joint, comprising a support arrangement (2) for supporting on a lower leg (112) and a retaining arrangement (3) for retention on a foot (110),
wherein a damping arrangement (4) is arranged with at least one damping element (40) for damping a relative movement between the support arrangement (2) and the retaining arrangement (3),
**characterized in that**
the damping arrangement (4) has a support attachment (42) for attaching to the support arrangement (2) and a retaining attachment (41) for attaching to the retaining arrangement (3),
wherein the support attachment (42) is connected to the support arrangement (2) at least partially on a first side (46) of the support arrangement (2), which, in a state attached to the lower leg (112), corresponds to a lateral side (L) of the ankle joint, and at least partially on a second side (47) of the support arrangement (2), which, in a state attached to the lower leg (112), corresponds to a medial side (M) of the ankle joint,
wherein the support attachment (42) has a first support arm (43) for attaching to the support arrangement (2) on the first side (46), and a second support arm (44) for attaching to the support arrangement (2) on the second side (47),
wherein the damping element (40) is integrated in the first support arm (43) and/or a damping element (40) is integrated in the second support arm (44),
**and in that** the support arrangement (2) comprises at least one supporting region (20, 21) and at least one supporting fixation (22), wherein the support arrangement (2) can be supported on the anklebone (114) with the at least one supporting region (20, 21) above the anklebone (114) of the ankle joint, and wherein the at least one supporting fixation (22) is configured for fixing the position of the at least one supporting region (20, 21) in a state attached to the lower leg (112).

2. The foot movement damping device (1) as claimed in claim 1, **characterized in that** the support arrangement (2) has at least one first supporting region (20) for supporting on a first side (46), preferably a lateral side (L) of the ankle joint, and at least one second supporting region (21) for supporting on a second side (47), preferably on a medial side (M) of the ankle joint.

3. The foot movement damping device (1) as claimed in claim 1 or 2, **characterized in that** the first support arm (43) and the second support arm (44) are formed integrally.

4. The foot movement damping device (1) as claimed in one of the preceding claims, **characterized in that** a damping element (40) is integrated in the retaining attachment (41).

5. The foot movement damping device (1) as claimed in claim 4, **characterized in that** the retaining attachment (41) together with a plane defined by a sole (30) of the retaining arrangement (3) encloses an angle of 0° to 90°, preferably of 30° to 70°, particularly preferably of 50° to 60° and very particularly preferably of 57°.

6. The foot movement damping device (1) as claimed in one of the preceding claims, **characterized in that** a transition (45) between the support attachment (42) and the retaining attachment (41) is configured in an articulated or movable manner.

7. A shoe (80) for damping a foot movement via the ankle joint, comprising a sole (82) and a top part, **characterized in that** the shoe (80) has a foot movement damping device (1) as claimed in one of the preceding claims.

8. The shoe (80) as claimed in claim 7, **characterized in that** the damping arrangement (4) is at least partially arranged on an outer side of the shoe (80).

9. The shoe (80) as claimed in claim 7 or 8, **characterized in that** the retaining attachment (41) is at least partially integrated in the shoe (80) and/or the support attachment (42) is at least partially integrated in the shoe (80) and/or at least one damping element (40) is integrated in the shoe (80), wherein preferably the entire damping arrangement (4) is integrated in the shoe (80).

10. The shoe (80) as claimed in one of claims 7 to 9, **characterized in that** the support arrangement (2) is provided as a separate component.

11. The shoe (80) as claimed in one of claims 7 to 9, **characterized in that** the shoe (80) has a high shaft (85), wherein the support arrangement (2) is integrated in the high shaft (85).

## Revendications

1. Dispositif d'amortissement du mouvement du pied (1) pour amortir un mouvement du pied par le biais de la cheville,
comprenant un agencement de support (2) pour s'appuyer sur un tibia (112) et un agencement de fixation (3) pour être fixé sur un pied (110),
dans lequel un agencement d'amortissement (4) avec au moins un élément d'amortissement (40) pour amortir un mouvement relatif est disposé entre l'agencement de support (2) et l'agencement de fixation (3),
**caractérisé en ce que**
l'agencement d'amortissement (4) présente une liaison de support (42) pour être relié sur l'agencement de support (2) et une liaison de fixation (41) pour être relié sur l'agencement de fixation (3), dans lequel la liaison de support (42) est relié au moins partiellement sur un premier côté (46) de l'agencement de support (2), lequel correspond dans un état monté sur le tibia (112) à un côté latéral (L) de la cheville, et au moins partiellement sur un second côté (47) de l'agencement de support (2), lequel correspond dans un état monté sur le tibia (112) à un côté médian (M) de la cheville,
dans lequel la liaison de support (42) présente un premier bras de support (43) pour être relié sur l'agencement de support (2) sur le premier côté, et un second bras de support (44) pour être relié sur l'agencement de support (2) sur le second côté (47),
dans lequel l'élément d'amortissement (40) est intégré dans le premier bras de support (43) et/ou un/l'élément d'amortissement (40) est intégré dans le second bras de support (44) ;
**et en ce que** l'agencement de support (2) comprend au moins une zone d'appui (20, 21) et au moins une fixation d'appui (22), dans lequel l'agencement de support (2) peut s'appuyer avec l'au moins une zone d'appui (20, 21) au-dessus de la malléole (114) de la cheville sur la malléole (114), et dans lequel l'au moins une fixation d'appui (22) pour fixer la position de l'au moins une zone d'appui (20, 21) est réalisée dans un état monté sur le tibia (112).

2. Dispositif d'amortissement du mouvement du pied (1) selon la revendication 1, **caractérisé en ce que** l'agencement de support (2) présente au moins une première zone d'appui (20) pour s'appuyer sur un premier côté (46), de préférence un côté latéral (L) de la cheville, et au moins une seconde zone d'appui (21) pour s'appuyer sur un second côté (47), de préférence un côté médian (M) de la cheville.

3. Dispositif d'amortissement du mouvement du pied (1) selon la revendication 1 ou 2, **caractérisé en ce que** le premier bras de support (43) et le second bras de support (44) sont réalisés d'un seul tenant.

4. Dispositif d'amortissement du mouvement du pied (1) selon l'une quelconque des revendications précédentes, **caractérisé en ce qu'un** élément d'amortissement (40) est intégré dans la liaison de fixation (41).

5. Dispositif d'amortissement du mouvement du pied (1) selon la revendication 4, **caractérisé en ce que** la liaison de fixation (41) inclut avec un plan défini par une semelle (30) de l'agencement de fixation (3) un angle allant de 0° à 90°, de préférence de 30° à 70°, le plus préférentiellement de 50° à 60° et le plus préférentiellement encore de 57°.

6. Dispositif d'amortissement du mouvement du pied (1) selon l'une quelconque des revendications précédentes, **caractérisé en ce qu'**un passage (45) entre la liaison de support (42) et la liaison de fixation (41) est réalisé de manière articulée ou mobile.

7. Chaussure (80) pour amortir un mouvement du pied par le biais de la cheville, comprenant une semelle (82) et une partie supérieure, **caractérisée en ce que** la chaussure (80) présente un dispositif d'amortissement du mouvement du pied (1) selon l'une quelconque des revendications précédentes.

8. Chaussure (80) selon la revendication 7, **caractérisée en ce que** l'agencement d'amortissement (4) est disposé au moins partiellement sur un côté extérieur de la chaussure (80).

9. Chaussure (80) selon la revendication 7 ou 8, **caractérisée en ce que** la liaison de fixation (41) est intégrée au moins partiellement dans la chaussure (80) et/ou la liaison de support (42) est intégrée au moins partiellement dans la chaussure (80) et/ou au moins un élément d'amortissement (40) est intégré dans la chaussure (80), dans laquelle de préférence la totalité de l'agencement d'amortissement (4) est intégrée dans la chaussure (80).

10. Chaussure (80) selon l'une quelconque des revendications 7 à 9, **caractérisée en ce que** l'agencement de support (2) est prévu comme composant distinct.

11. Chaussure (80) selon l'une quelconque des revendications 7 à 9, **caractérisée en ce que** la chaussure (80) présente une tige élevée (85), dans laquelle l'agencement de support (2) est intégré dans la tige élevée (85).
